# EUROPEAN PATENT SPECIFICATION

(11) **EP 3 138 913 B1**
(45) Date of publication and mention of the grant of the patent: **22.05.2019**
(21) Application number: 16186708.0
(22) Date of filing: 31.08.2016
(51) Int. Cl.: C12N 15/10, C12Q 1/68

(54) **METHOD AND KIT FOR SELECTIVE ISOLATION OF NUCLEIC ACID**
VERFAHREN UND KIT ZUR SELEKTIVEN ISOLIERUNG VON NUKLEINSÄURE
PROCÉDÉ ET KIT D'ISOLEMENT SÉLECTIF D'ACIDE NUCLÉIQUE

(30) Priority: 02.09.2015 TW 104129133
(43) Date of publication of application: 08.03.2017
(73) Proprietor: Imagen Bioscience, Taipei City 114 (TW)
(72) Inventor: Lo, Yuan-Ting, Taipei City 114 (TW); Chung, Ting-Hao, Taichung City 404 (TW)
(74) Representative: Lang, Christian

(56) References cited:
- EP-A2- 0 818 461
- WO-A1-2004/108925
- WO-A1-2013/045432
- WO-A1-2013/045434
- WO-A1-2013/181651
- WO-A2-2007/140417

## Description

### FIELD OF THE INVENTION

The present invention is related to a technology of nucleic acid isolation, especially related to a method and a kit for selective isolation of nucleic acid.

### BACKGROUND OF THE INVENTION

With gradual decoding of human genome and other species genome, molecular diagnosis technology that uses nucleic acid (including DNA and RNA) as a detection target has been a key point for clinic detection. Nucleic acid, no matter DNA or RNA, is consisted of four nucleotide molecules. Thus, the compositions of nucleotide are relatively simple and easily detected, when compared with complicated clinic pathological interpretation.

The size of nucleic acid fragment plays a very important role on selection or filtration during molecular diagnosis that uses nucleic acid as a target.

For example, cell-free nucleic acid in blood system is often used as a main source of nucleic acid for prenatal detection of fetal and cancer detection. But most cell-free nucleic acids are fragments of short sequences and the amount thereof is relatively fewer when compared with other bigger nucleic acids. Accordingly, how to get enough nucleic acids of short fragments for is an important factor on determining accuracy of detection results.

On the other hand, recently next generation sequencing (NGS) is a main tool for nucleic acid sequencing in molecule diagnosis technology that uses nucleic acid as a target, because it does not do plastid replication with bacteria, has high flux and spends short time. Take NGS of Illumina system as an example, nucleic acid to be tested is segmented first, and then the nucleic acid fragments in different sizes are filtrated and the fragments of short sequences are purified according to requirements, recovered, concentrated, or treated by polymerase chain reaction (PCR) for amplification to build nucleic acid database. Next, the nucleic acid fragments in nucleic acid database is coupled to conjugating sequences, and the conjugated fragments are injected into a chip on which the complementary sequences with respect to the conjugating sequences are attached for doing bridge PCR and fluorescent marking and detection. The filtration of nucleic acid fragments may be a pretreatment for building the nucleic acid database, remove nucleic acid fragments that do not have ready conjugation with the conjugating sequences and are not targets after PCR amplification, which may improve the quality and amount of nucleic acid during sequencing.

For the foregoing filtration of nucleic acid fragments, the recovered target nucleic acid fragments substantially is usually not long, and the recovered target nucleic acid fragments is called "small-size fragments" in this technology field. Accordingly, how to improve the recovery effect of small-size nucleic acid fragments, in order to maintain and strengthen the quality of sequencing, there are several method which are developed, which includes three types: (1) the nucleic acid of different fragments size is distinguished by agarose gel electrophoresis. Next, the target fragments (usually are small-size nucleic acid fragments) is isolated and recovered by gel extraction. With the gel extraction method, the nucleic acid fragment of a specific size can be recovered accurately but the time consuming is an issue. In addition, due to the small-size nucleic acid fragments is limited to smaller molecular weight, the resolution in the agarose gel is inferior, and the recovery effect of the gel extraction is easily interfered at the low concentration. (2) By using the magnetic beads with the coated DNA molecular and surface functional group modification (for example, with a carboxyl group), with the concentration of polyethylene glycol (PEG) and salts, the magnetic beads absorbs and binding the small-size nucleic acid fragment which is to be isolated, as published in U.S. Patent No. 6,534,262. The advantage of above method is that can easily be applied to the automation system to isolate and filter a large number of the nucleic acid fragments. But the specific modified magnetic beads selected cause additional costs and processes. However, this approach is not applied for the nucleic acid with longer fragment size (for example, the fragment length is larger than about 400 bp). This approach significantly limits the application of technology in the NGS. (3) The primer or bait recovered the nucleic acid accurately. Unfortunately, this approach merely applied for the target nucleic acid fragment is known to be sequenced which is difficult to use widely.

According to the above problem is not resolved by conventional approach, the isolating or recovering for the nucleic acid fragments still continue to improve currently, but some approaches still have limitations. As illustrated as below:

U.S Patent No. 6,534,262B1 disclosed that by adjusting the conditions (such as concentration) of PEG and is associated with a solid carrier having a functional group, to adjust the ionic strength of the reaction solution and the concentration of PEG, to improve the recovering effect of small nucleic acid fragments. For example, in U.S Publication Patent No. 20060024701 and U.S. Publication Patent 2011060135, utilizes the adjusting method by adjusting PEG condition to reach the isolation or recovery of small-size nucleic acid fragments which is associated with the functional (carboxyl or amine) modified solid carrier (films, particles, beads), so that the cost and complexity of manufacture are increased.

Application patent No. WO2013181651A1 disclosed a method for recovering small-size nucleic acid fragments, which disclosed that the guanidine salt concentration and pH value is adjusted to adjust the binding effect of small nucleic acid fragment on the solid carrier, such that the recovery effect is to be improved. However, the application timing for the filtration of NGS sequences size is diversified, the nucleic acids recovery bound to face diverse and complex solution environment, and it might introduce the changes in pH value so as to the effect and reliability of this method is decreased. Further, during the production of several related solutions reagent, the standard requirements for the accuracy of the pH value is relatively increased, and even the inspection process needs to be increased, such that the time is consumed and the manufacturing cost is increased. In addition, U.S. Publication Patent 20140243216 utilizes the strategy with adjusting pH value to achieve the purpose of the recovery of small-size nucleic acid fragments, and therefore, in accordance with its dependence on pH value, the method disclosed in U.S. Publication Patent 20140243216 that can be expected to the different environment solutions might cause the adverse effect.

From WO 2013/181651 A1, WO 2004/108925 A1, WO 2013/045434 A1, WO 2013/045432 A1, WO 2007/140417 A2 and EP 0818461 A2 different methods are known by means of which nucleic acid fragments of different size contained in a solution can be separated, wherein the actual separation is achieved by binding fragments of a certain size fraction to a solid phase. Different size fractions are bound to the solid phase by adjusting the salt concentrations and/or the pH values of the binding buffers.

### SUMMARY OF THE INVENTION

In order to solve the above disadvantages of the conventional prior art, the present invention aims to provide a method and kit for selective isolation of nucleic acid to isolate the nucleic acids of specific fragment.

It is an objective of the present invention to provide a method and kit for selective isolation of nucleic acid including a simple reaction buffer solution, so that the nucleic acid fragments of specific size can be easily isolated from a nucleic acid mixing solution having different fragments therein according to the requirement of the nucleic acid fragments of specific size for the user.

It is a further objective of the present invention to provide a method and kit for selective isolation of nucleic acid allowing a user to selectively isolate the nucleic acid fragments of specific size (large-size fragment or small-size fragment) and applied directly to a subsequent process, not only quick and easy, low cost, recovery is also good, and the subsequent application efficacy is improved.

It is another objective of the present invention to provide a method for isolating a nucleic acid using a solution environment that is simple and stable, wherein purification and isolation effects of the nucleic acid being affected by changes in pH value can be excluded. Further, the method for selective isolation of nucleic acid of the present invention did not rely on a particular polymer solute or surfactant, such as polyethylene glycol (PEG) solution system, and even without the use of specific modified functional groups (such as surface carboxylation or amination) modified solid carrier and therefore the solution used in the method for selective isolation of nucleic acid of the present invention is suitable for the existing automation systems for high-throughput of the operation, while the precision, isolation, and purification efficiency of nucleic acid can also be satisfied.

It is a further objective of the present invention to provide a method for selective isolation of nucleic acid utilizing simple ingredients of salt solution and crowding agent with silicon-based reagent (silica-based) filter to achieve the isolation of the nucleic acid o fragment of a specific size.

It is another objective of the present invention to provide a method for selective isolation of nucleic acid using a simple formulation, without using the polymer solvent, without using a functional group of the modified filter, and is also did not need to rely on pH values, especially for isolating the nucleic acid fragments of specific size.

According to the invention, to achieve the above objectives, a first method for selective isolation of nucleic acid is provided which comprises the features of claim 1. According to the invention also a second method is provided which comprises the features of claim 3. In addition, a kit for selective isolation of nucleic acid is provided that comprises the features of claim 5. Said first method comprises: mixing a nucleic acid sample and a binding buffer solution to form a mixing solution, wherein the nucleic acid sample has a nucleic acid fragment of a specific size, and the mixing solution has a chaotropic salt and one or two of a monovalent salt or a crowding agent, wherein the chaotropic salt is guanidine hydrochloride (GuHCl), guanidine thiocyanate (GuSCN), sodium iodide (NaI) or sodium perchlorate (NaClO₄), wherein the monovalent salt is lithium chloride (LiCl), lithium acetate (LiOAc), sodium acetate (NaOAc), potassium acetate (KOAc) or ammonium acetate (NH₄OAc), wherein the crowding agent is ethanol, isopropanol or polyethylene glycol, and wherein, when the monovalent salt concentration is high, the large-size nucleic acid fragment is bound to a solid carrier and when the concentration of monovalent salt is low, the bigger range (includes large-size and small size) of nucleic acid fragments is bound to the solid carrier; passing the mixing solution through a solid carrier to make the nucleic acid fragment of the specific size bind to the solid carrier;; passing a washing buffer solution through the solid carrier to remove subject that does not bind to the solid carrier; and passing an aqueous solution through the solid carrier to recovery a reserving nucleic acid fragment of the specific size, whereby changes of pH value of the mixing solution is less than or equal to 0,4, if the concentration of guanidine hydrochloride (GuHCl) is changed from 0.6M to 4M or the concentration of guanidine thiocyanate (GuSCN) is changed from 0.6M to 4M or the concentration of sodium iodide (NaI) is changed from 2.5M to 3.5M or the concentration of sodium perchlorate (NaClO₄) is changed from 2.5M to 3.5M, whereby changes of pH value of the mixing solution is less than or equal to 0,4, if the concentration of guanidine hydrochloride (GuHCl) is changed from 0.6M to 4M or the concentration of guanidine thiocyanate (GuSCN) is changed from 0.6M to 4M or the concentration of sodium iodide (NaI) is changed from 2.5M to 3.5M or the concentration of sodium perchlorate (NaClO₄) is changed from 2.5M to 3.5M.

To achieve the above objectives, another method for selective isolation of nucleic acid is provided, which comprises: mixing a nucleic acid sample and a first binding buffer solution to form a first mixing solution, wherein the nucleic acid sample has a nucleic acid fragment of a specific size, and the first mixing solution has a chaotropic salt, wherein the chaotropic salt is guanidine hydrochloride (GuHCl), guanidine thiocyanate (GuSCN), sodium iodide (NaI) or sodium perchlorate (NaClO₄); passing the first mixing solution through a first solid carrier to acquire a once filtrated liquid, wherein the once filtrated liquid comprises the nucleic acid fragment of the specific size; mixing the once filtrated liquid and a second binding buffer solution to form a second mixing solution, wherein the second mixing solution has the chaotropic salt, and one or two of a monovalent salt or a crowding agent, wherein the monovalent salt is lithium chloride (LiCl), lithium acetate (LiOAc), sodium acetate (NaOAc), potassium acetate (KOAc) or ammonium acetate (NH₄OAc), wherein the monovalent salt is lithium chloride (LiCl), lithium acetate (LiOAc), sodium acetate (NaOAc), potassium acetate (KOAc) or ammonium acetate (NH₄OAc), wherein the crowding agent is ethanol, isopropanol or polyethylene glycol; passing a second mixing solution through a second solid carrier to make the nucleic acid fragment of specific size bind to the second solid carrier, wherein when the concentration of monovalent salt is high, the large-size nucleic acid fragment is bound to the solid carrier and when the concentration of monovalent salt is low, the bigger range (includes large-size and small size) of nucleic acid fragment is bound to the solid carrier; passing a washing buffer liquid through the second solid carrier to remove subject that does not bind to the second solid carrier; and passing an aqueous solution through the second solid carrier to recovery a preserving nucleic acid fragment of the specific size, whereby changes of pH value of the mixing solution is less than or equal to 0,4, if the concentration of guanidine hydrochloride (GuHCl) is changed from 0.6M to 4M or the concentration of guanidine thiocyanate (GuSCN) is changed from 0.6M to 4M or the concentration of sodium iodide (NaI) is changed from 2.5M to 3.5M or the concentration of sodium perchlorate (NaClO₄) is changed from 2.5M to 3.5M..

Accordingly, a kit for selective isolation of nucleic acid is provided, which comprises: at least a binding buffer solution including a chaotropic salt, and at least one of a monovalent salt or a crowding agent; and at least a solid carrier, the characteristic is in that: the chaotropic salt is guanidine hydrochloride (GuHCl), guanidine thiocyanate (GuSCN), sodium iodide (NaI) or sodium perchlorate (NaClO₄), wherein the monovalent salt is lithium chloride (LiCl), lithium acetate (LiOAc), sodium acetate (NaOAc), potassium acetate (KOAc) or ammonium acetate (NH₄OAc), wherein the crowding agent is ethanol, isopropanol or polyethylene glycol, and, when the concentration of chaotropic salt is fixed, the concentration of crowding agent is low, or the concentration of monovalent salt is higher than 0.208 M and the large-size nucleic acid fragment that is bound to the solid carrier is bigger than 300 bp or 2000 bp, or the concentration of monovalent salt is 0.625 M and the large-size nucleic acid fragment that is bound to the solid carrier is bigger than 1000 bp or 5000 bp, and when the monovalent salt concentration is lower than 0.208 M, the bigger range which includes large-size and small-size of nucleic acid fragments are bound to the solid carrier, whereby a mixing solution is formed by mixing the binding buffer solution and the nucleic acid, and changes of pH value of the mixing solution is less than or equal to 0.4, if the concentration of guanidine hydrochloride (GuHCl) is changed from 0.6M to 4M or the concentration of guanidine thiocyanate (GuSCN) is changed from 0.6M to 4M or the concentration of sodium iodide (NaI) is changed from 2.5M to 3.5M or the concentration of sodium perchlorate (NaClO₄) is changed from 2.5M to 3.5M.

For being understood by the one having general knowledge in this field, some embodiments of the present invention are described herein. Some technology content well known by the one having the general knowledge will not be repeatedly illustrated in the disclosure. However, the embodiments of the present invention are only used to illustrate some preferred implementations of the present invention but not to limit the scope of the attached claims.

### BRIEF DESCRIPTION OF THE DRAWINGS

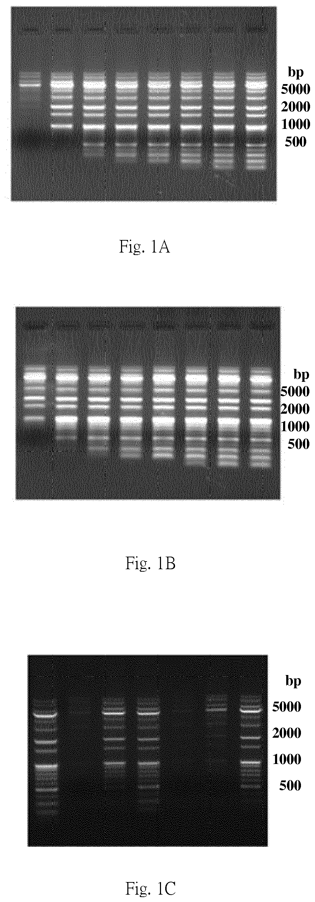

Fig. 1A to Fig. 1C respectively show the isolation degrees of nucleic acid fragments after DNA standard solutions are treated by chaotropic salts in different concentrations and types. From column 1 to column 7 in Fig. 1A are results after treatments shown in Table 1-1 lanes 1-7, respectively; from column 8 to column 14 in Fig. 1B are the ones after treatments shown in Table 1-2 lanes 8-14, respectively; from column 15 to column 17 in Fig. 1C are the ones after treatments shown in Table 1-3 lanes 15-17, and column 18 to column 20 are the ones after treatments shown in Table 1-4 lanes 18-20, respectively; and columns M in Fig. 1A-Fig. 1C is DNA standard solutions without treatments.

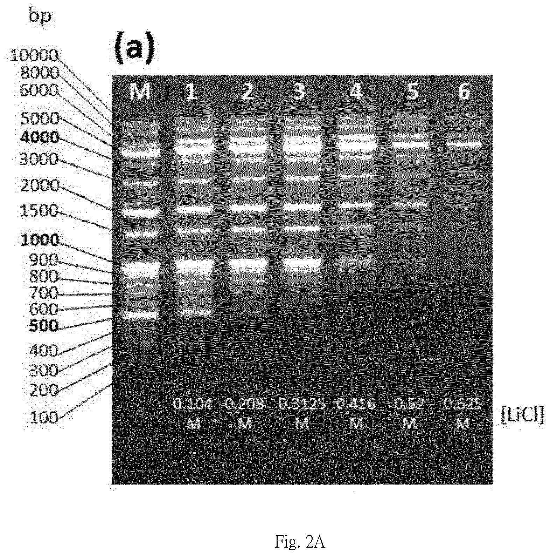

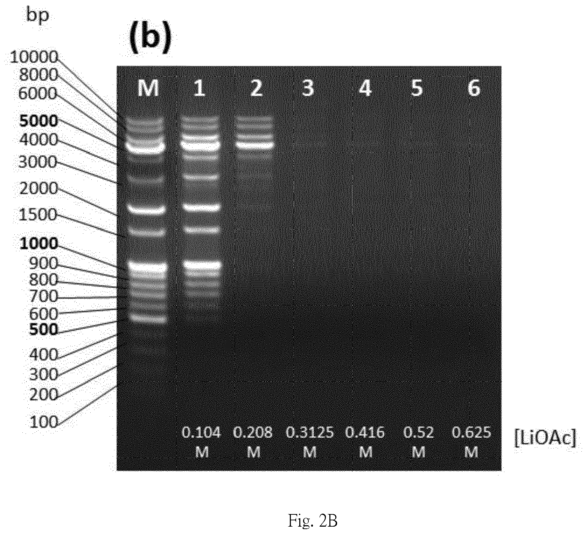

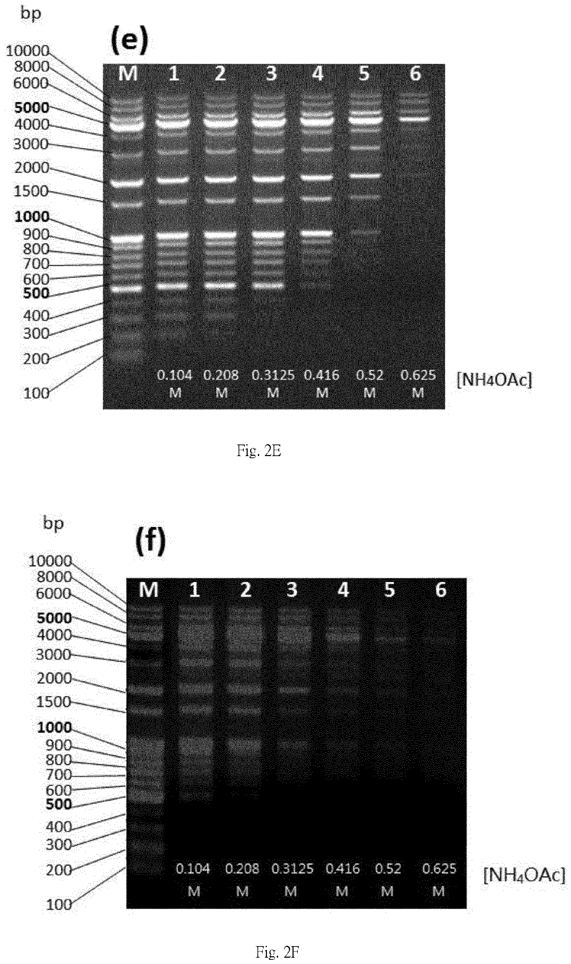

Fig. 2A to Fig. 2E respectively show the isolation degrees of nucleic acid fragments after DNA standard solutions are treated by chaotropic salts of same concentration in addition to monovalent salts in different concentrations and types. From column 1 to column 7 in Fig. 2A are results after treatments shown in Table 2-1 lanes 1-7, respectively, respectively; from column 1 to column 7 in Fig. 2B-Fig. 2E is the ones after treatments shown in Table 2-3 lanes 1-7, respectively; from column 1 to column 7 in Fig. 2F are the ones after treatments shown in Table 2-8 lanes 1-7, respectively; and columns M in Fig. 2A-Fig. 2E is DNA standard solutions without treatments.

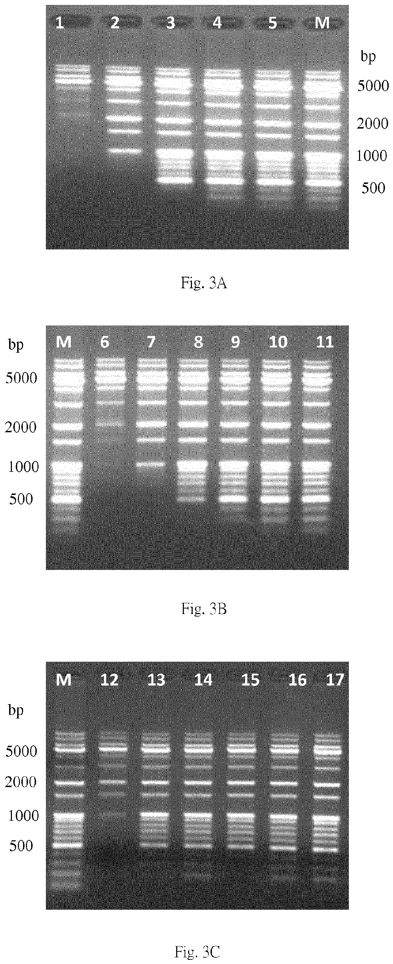

Fig. 3A to Fig. 3C respectively show the isolation degrees of nucleic acid fragments after DNA standard solutions are treated by chaotropic salts of same concentration in addition to crowding agents in different concentrations. From column 1 to column 5 in Fig. 3A are results after treatments shown in Table 3-1 lanes 1-5, respectively; from column 1 to column 5 in Fig. 3B are the ones after treatments shown in Table 3-2 lanes 6-10; from column 1 to column 3 in Fig. 3C are the ones after treatments shown in Table 3-3 lanes 1-3, respectively, and column 4 to column 6 is the ones after treatments shown in Table 3-4 lanes 4-6, respectively; and columns M in Fig. 3A-Fig. 3C is DNA standard solutions without treatments.

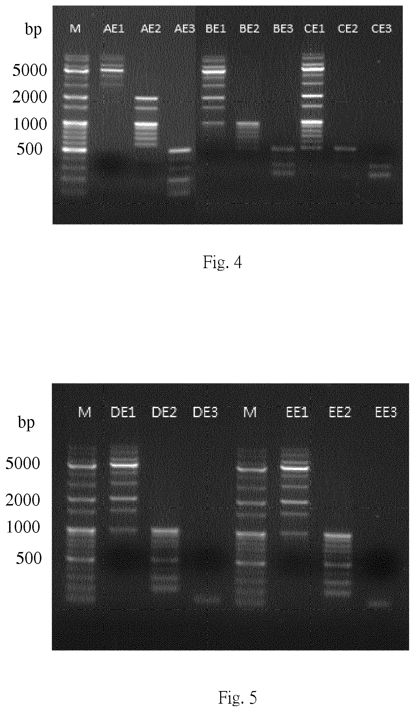

Fig. 4 shows the isolation degrees of nucleic acid fragments after DNA standard solutions are filtrated once to third times by various mixtures of chaotropic salt, monovalent salt and crowding agent associated with a solid carrier of spin column made of silica material. From column AE1 to column CE3 are results after treatments shown in Table 4-1 lanes AM1-CM3, respectively, and columns M is DNA standard solution without treatments.
Fig. 5 shows the isolation degrees of nucleic acid fragments after DNA standard solutions are filtrated once to third times by various mixtures of chaotropic salt, monovalent salt and crowding agent associated with a solid carrier of magnetic beads. From column DE1 to column EE3 are results after treatments shown in Table 4-2 lanes DM1-EM3, respectively, and columns M is DNA standard solution without treatments.

### DETAILED DESCRIPTION OF THE PREFERRED EMBODIMENTS

The method and kit for the selective isolation of nucleic acid in the present invention help operators easily isolate nucleic acid fragment of specific size from a nucleic acid sample including the ones of various fragment size, according to their requirement on predetermined nucleic acid fragments. That is, the method and the kit in the present invention enable operators selectively isolate nucleic acid fragment of specific size and directly utilize it to other subsequent processes, which may improve subsequent application effects.

Besides, solution environment used in the method and the kits in the present invention is simple and stable without the control of pH values for the solution environment and the dependence of a carrier with specific modified functional groups (such as surface carboxylation or amination), which is suitable to apply to current automation systems to have high throughput operation.

Just by using chaotropic salt, monovalent salt and crowding agent of different concentrations to directly influence the retention of nucleic acid and achieve staged filtration or retention, the present invention provides the selective isolation of nucleic acid with the simple, easy operation, broad application, easy adjustment on solution conditions, and low cost method and kit.

The term "Nucleic acid" in the present disclosure includes but not limits to the ones from synthetic, nature, or mixing of synthetic and nature sources. The sizes, lengths or dimensions of fragment include the ones of nature or synthetic nucleic acid of single strand, double strands or complementary sequences, or the amount of nucleic acid unit for nucleic acid sequence consisted of DNA or RNA. The nucleic acid unit is a counting unit of nucleic acid sequence, and the counting unit often used includes base pair or nucleotide molecule. Base means a nucleic acid unit. In order to explain, the terms of large-size fragment, and small-size fragment to describe only under certain exemplary conditions, and is distinguished by a relatively common general technical knowledge of longer or shorter nucleic acid fragment, and it is not intended to limit the fragment-size of the specific nucleic acid. This is well known to those skilled in the art and did not describe herein. In addition, the purification material for the nucleic acid, the technical principles, characteristics, equipment, the basic operation flow, has been understand in the related art, and it does not further describe hereinafter.

The term "chaotropic salt" in the present disclosure refers to a substance that may influence the arrangement of water molecules in an aqueous solution or change the strength of hydrogen bond within molecules of protein or nucleic acid. The chaotropic salt used in the method and kit according to the present invention includes, but not limit to, guanidine thiocyanate (GuSCN), guanidine hydrochloride (GuHCl), sodium iodide (NaI) or sodium perchlorate (NaClO₄). The chaotropic salt in different concentrations performs different selection or filtration on the sizes of the nucleic acid fragments. Generally, the chaotropic salt in low concentration only keeps the larger nucleic acid fragments within the solid carrier and filtrate the smaller ones into a flow-through. With the increase of the concentrations, the chaotropic salt of the present invention can gradually keep the smaller nucleic acid fragments on the solid carrier.

The term "monovalent salt" in the present disclosure refers to an ionic compound with monovalent cation and anion. The monovalent salt used in the method and kit according to the present invention includes chloride salt of monovalent cation and acetate salt of monovalent cation. The chloride salt of monovalent cation includes, but not limit to, LiCl, NaCl, KCl and NH₄Cl, wherein preferably LiCl is used in the method and kit according to the present invention. The acetate salt of monovalent cation for the method and kit according to the present invention includes Lithium acetate (LiOAc), sodium acetate (NaOAc), potassium acetate (KOAc) and ammonium acetate (NH₄Oac). The selectivity on the sizes of nucleic acid fragments can be achieved by coordinating monovalent salt with the chaotropic salt to be measured by LiOAc, NaOAc, KOAc and NH₄OAc. The role of monovalent salt in different concentrations on the size selectivity of nucleic acid fragment can be understood by experiments. Generally, monovalent salt in low concentrations may preserve small-size nucleic acid fragments in solid carrier. With the increase of the concentrations of the monovalent salt, the monovalent salt has weak control on small-size nucleic acid fragments, and consequently, only larger-size nucleic acid fragments are kept in solid carrier but the small-size ones may be left in flow-through.

The term "crowding agent" in the present disclosure refers to an agent that can occupy most volume in a solution and obligate solvents to aggregate or concentrate within small volume. The crowding agent includes monols, polyols and large-molecule polymer. The crowding agent in the method and kit according to the present invention may be ethanol, isopropanol and polyethylene glycol (PEG). According to the present invention, ethanol, isopropanol and PEG are used to measure the size selectivity of nucleic acid fragment achieved by associating the crowding agent with the chaotropic salt. The crowding agent in low concentration only may preserve the large-size nucleic acid fragments in the solid carrier and leave the small-size ones in the flow-through. With the increase of the concentration, the crowding agent aforementioned gradually has stronger control on keeping the small-size nucleic acid fragments in the solid carrier.

The following examples will be discussed with reference to the drawings to illustrate the technical contents and characteristics of the methods and kits according to the present invention.

The first Experiment discusses the influences of chaotropic salts in various types and various concentrations over the sizes of selected nucleic acid fragment.

A DNA standard solution for tests is a mixture of the first 20µL 1kb DNA (Sharp DNA Ladder Markers 1kb, ELPIS Biotech, Korea) and the second 20µL 100 bp DNA (Sharp DNA Ladder Markers 100bp, ELPIS Biotech, Korea). Shown in Table 1-1 to Table 1-4 as follows, a mixing solution is acquired by mixing the 40µL DNA standard solution for tests with a buffer solution in 200µL. And measurements are made under guanidine hydrochloride (GuHCl) in concentration from 0.6M to 4M and final volume of 200µL, guanidine thiocyanate (GuSCN) in concentration from 0.6M to 4M and final volume of 200µL, sodium iodide (NaI) in concentration from 2.5M to 3.5M and final volume of 200µL, and sodium perchlorate (NaClO₄) in concentration from 2.5M to 3.5M and final volume of 200µL. The pH values of the mixing solutions under the conditions aforementioned are from 5.44 to 5.84, from 5.59 to 5.73, from 6.25 to 6.40, and from 6.66 to 6.57, respectively. It can be obtained that pH value of the mixing solution does not have significant changes with the different concentration level of chaotropic salts. Next, each of the mixing solutions is added into spin column (MinElute® Qiagen) wihich is made of silica material, and hereinafter is described as spin column, and centrifuged in 13000 rpm for one minute for bind of DNA and solid carrier within the spin column. Next, 60%, 500ml alcohol as a washing buffer solution is added into the spin column after centrifugation, and is centrifuged in 13000 rpm for one minute for removal of DNA fragments that do not bind with the spin column. The DNA fragments may be isolated from the spin column in which 40 ml aqureous solution is added by centrifuging in 13000 rpm for one minute to acquire a final product. The products from nucleic acid filtration under the aforementioned concentration conditions are treated by gel electrophoresis to have results shown in Fig. 1A, and from Table 1-1 to Table 1-4. According to above experiment, when the concentration of chaotropic salts is low, only large range nucleic acid fragment preserved on the the spin column (solid carrier), and the small-size nucliec acid fragment is filtered into the flow-through. It is should be illustrated that the large range nucleic acid fragment may include large-szie fragement, middle-size fragment and/or small-size fragment. With the increase of the concentration of chaotropic salt, the chaotropic salt can preserve the small-size nucleic acid fragment on spin column (solid carrier), that is, the large-size nucleic acid fragment is to be isolated (or filtered) into the flow-through.

Fig. 1A shows the isolation degrees of nucleic acid fragments under GuHCl in different concentrations. Depicted in Fig. 1A, the size of nucleic acid fragment larger than 4000 bp may be filtrated out in 0.6M GuHCl. That is, the size of nucleic acid fragments smaller than 4000 bp can be eliminated because they do not bind the solid carrier. Next, the size of nucleic acid fragment larger than 1000 bp may be filtrated out in 0.7M GuHCl. That is, the size of nucleic acid fragments smaller than 1000 bp can be eliminated because they do not bind the solid carrier. Next, the size of nucleic acid fragment larger than 300 bp may be filtrated out in 0.8M GuHCl. That is, size of the nucleic acid fragments smaller than 300 bp can be eliminated because they do not bind the solid carrier. Next, the nucleic acid fragment larger than 200 bp may be filtrated out in 0.9M GuHCl. That is, the size of nucleic acid fragments smaller than 200 bp can be eliminated because they do not bind the solid carrier. Next, the size of nucleic acid fragment larger than 200 bp may be filtrated out in 1M GuHCl. That is, the size of nucleic acid fragments smaller than 200 bp can be isolated because they do not bind the solid carrier. Next, the size of nucleic acid fragment larger than 100 bp may be filtrated out in 2M GuHCl and 4M GuHCl. That is, the nucleic acid fragments smaller than 100 bp can be eliminated because they do not bind the solid carrier. The associations between the concentrations of guanidine hydrochloride (GuHCl) and the filtration of the nucleic acid fragment are shown in Table 1-1.

**Table 1-1: the associations between the concentrations of guanidine hydrochloride (GuHCl) and the sizes of the nucleic acid fragment**

| condition | Concentration of GuHCl | pH value of final mixing solution | Sizes of preserved nucleic acid fragments |
|---|---|---|---|
| 1 | 0.6M | 5.84 | more than 4000 bp |
| 2 | 0.7M | 5.63 | more than 1000 bp |
| 3 | 0.8M | 5.53 | more than 300 bp |
| 4 | 0.9M | 5.51 | more than 200 bp |
| 5 | 1.0M | 5.71 | more than 200 bp |
| 6 | 2.0M | 5.60 | more than 100 bp |
| 7 | 4.0M | 5.44 | more than 100 bp |

Fig. 1B shows the isolation degrees of nucleic acid fragments under guanidine thiocyanate (GuSCN) in different concentrations. Depicted in Fig. 1B, the size of nucleic acid fragment larger than 1000 bp may be filtrated out in 0.6M GuSCN. That is, the size of nucleic acid fragments smaller than 1000 bp can be eliminated because they do not bind the solid carrier. Next, the size of nucleic acid fragment larger than 500 bp may be filtrated out in 0.7M GuSCN. That is, the size of nucleic acid fragments smaller than 500 bp can be eliminated because they do not bind the solid carrier. Next, the size of nucleic acid fragment larger than 200 bp may be filtrated out in 0.8M, 0.9M, and 1M GuSCN. That is, the size of nucleic acid fragments smaller than 200 bp can be eliminated because they do not bind the solid carrier. Next, the size of nucleic acid fragment larger than 100 bp may be filtrated out in 1.5M and 3M GuSCN. That is, the size of nucleic acid fragments smaller than 100 bp can be eliminated because they do not bind the solid carrier. The associations between the concentrations of GuSCN and the filtration of the nucleic acid fragment are shown in Table 1-2.

**Table 1-2: the associations between the concentrations of GuSCN and the sizes of the filtrated nucleic acid fragment**

| condition | Concentration of GuSCN | pH value of final mixing solution | Sizes of preserved nucleic acid fragment |
|---|---|---|---|
| 8 | 0.6M | 5.71 | more than 1000 bp |
| 9 | 0.7M | 5.62 | more than 500 bp |
| 10 | 0.8M | 5.60 | more than 200 bp |
| 11 | 0.9M | 5.62 | more than 200 bp |
| 12 | 1.0M | 5.68 | more than 200 bp |
| 13 | 1.5M | 5.59 | more than 100 bp |
| 14 | 3.0M | 5.73 | more than 100 bp |

Table 1-1 and Table 1-2 list the selection of chaotropic salt with different concentration and the size of nucleic acid fragment, in which the above listed data reflect the selection of chaotropic salts in different concentrations (guanidine hydrochloride and guanidine thiocyanate) for nucleic acid fragment of specific size.

Fig. 1C shows the isolation degrees of nucleic acid fragments under NaI in different concentrations. Depicted in Fig. 1C, there is no explicit nucleic acid to be kept by the solid carrier in 2.5M NaI. Next, the size of nucleic acid fragment larger than 500 bp may be filtrated out in 3M NaI. That is, the size of nucleic acid fragments smaller than 500 bp can be eliminated because they do not bind the solid carrier. Next, the size of nucleic acid fragment larger than 200 bp may be filtrated out in 3.5M NaI. That is, the size of nucleic acid fragments smaller than 200 bp can be eliminated because they do not bind the solid carrier. The associations between the concentrations of NaI and the filtration of the nucleic acid fragment are shown in Table 1-3.

**Table 1-3: the associations between the concentrations of NaI and the sizes of the filtrated nucleic acid fragment**

| condition | Concentration of NaI | pH value of final mixing solution | Sizes of preserved nucleic acid fragment |
|---|---|---|---|
| 15 | 2.5M | 6.36 | -- |
| 16 | 3M | 6.25 | more than 500 bp |
| 17 | 3.5M | 6.40 | more than 200 bp |

FIG. 1C shows the isolation degrees of nucleic acid fragments under sodium perchlorate (NaClO₄) in different concentrations. Depicted in Fig. 1C, there is no explicit nucleic acid to be kept by the solid carrier in 2.5M NaClO₄. Next, the size of nucleic acid fragment larger than 1000 bp may be filtrated out in 3M NaClO₄. That is, the size of nucleic acid fragments smaller than 1000 bp can be eliminated because they do not bind the solid carrier. Next, the size of nucleic acid fragment larger than 500 bp may be filtrated out in 3.5M NaClO₄. That is, the size of nucleic acid fragments smaller than 500 bp can be eliminated because they do not bind the solid carrier. The associations between the concentrations of NaClO₄ and the filtration of the nucleic acid fragment are shown in Table 1-4.

**Table 1-4: the associations between the concentrations of NaClO₄ and the sizes of the filtrated nucleic acid fragments**

| condition | Concentration of NaClO₄ | pH value of final mixing solution | Sizes of preserved nucleic acid fragment |
|---|---|---|---|
| 18 | 2.5M | 6.57 | -- |
| 19 | 3M | 6.66 | More than 1000 bp |
| 20 | 3.5M | 6.58 | More than 500 bp |

From the results of the first experiment, more and more smaller size of nucleic acid fragments to be caught increase with the concentrations of chaotropic salt, no matter GuSCN, GuHCl, NaI or NaClO₄, and vice versa. Furthermore, by actually measuring the pH values of the mixing solutions, the pH values of the mixing solutions are found to be varied within a narrow range. In this regard, the characteristic that the different sizes of nucleus acid fragments can be selectively filtrated by the chaotropic salts in various concentrations does not correlate with the pH values of the mixing solutions.

The second experiment discusses the influences of same chaotropic salts and monovalent salts in various types and various concentrations over the sizes of selected nucleic acid fragment.
1. Lithium chloride (LiCl) in different concentrations is added into guanidine thiocyanate (GuSCN).

The DNA standard solutions for tests are prepared according to the method of the first experiment. After completed mixing of 40µL DNA standard solution and respective 200µL binding buffer solutions in different conditions, the filtration of the nucleus acids is processed by the first experiment. The compositions of the final mixing solution are shown in Table 2-1. The final concentration of GuSCN is fixed to be 0.8333M and respectively added with 0.104M, 0.208M, 0.3125M, 0.416M, 0.52M, and 0.625M LiCl.

**Table 2-1 the compositions in final mixing solutions**

| condition | Concentration of GuSCN | Concentration of LiCl |
|---|---|---|
| 1 | 0.833M | 0.104M |
| 2 | 0.833M | 0.208M |
| 3 | 0.833M | 0.3125M |
| 4 | 0.833M | 0.416M |
| 5 | 0.833M | 0.52M |
| 6 | 0.833M | 0.625M |

The products from nucleic acid filtration under the binding buffer solutions added with the compositions from condition 1 to condition 6 are treated by gel electrophoresis to have results shown in Fig. 2A, and Table 2-2.

Fig. 2A shows the isolation degrees of nucleic acid fragments under 0.833M GuSCN and LiCl in different concentrations as the binding buffer solutions. The pH values of the final mixing solution under various conditions are measured to be from 5.82 to 5.88. Depicted in Fig. 2A, the size of nucleic acid fragment larger than 500 bp may be filtrated out in 0.104M, 0.208M, and 0.3125M LiCl. That is, the size of nucleic acid fragments smaller than 500 bp can be eliminated because they do not bind the solid carrier. Next, the size of nucleic acid fragment larger than 1000 bp may be filtrated out in 0.416M and 0.52M LiCl. That is, the size of nucleic acid fragments smaller than 1000 bp can be eliminated because they do not bind the solid carrier. Next, the size of nucleic acid fragment larger than 2000 bp may be filtrated out in 0.625M LiCl. That is, the size of nucleic acid fragments smaller than 2000 bp can be eliminated because they do not bind the solid carrier. The associations between the concentrations of lithium chloride (LiCl) and the filtration of the nucleic acid fragment are shown in Table 2-2.

**Table 2-2: the associations between the concentrations of LiCl and the sizes of the filtrated nucleic acid fragments**

| Conc. of GuSCN | Conc. Of LiCl | pH value of final mixing solution | Sizes of preserved nucleic acid fragment |
|---|---|---|---|
| 0.833M | 0.104M | 5.84 | more than 500 bp |
| 0.833M | 0.208M | 5.83 | more than 500 bp |
| 0.833M | 0.3125M | 5.85 | more than 500 bp |
| 0.833M | 0.416M | 5.88 | more than 1000 bp |
| 0.833M | 0.52M | 5.82 | more than 1000 bp |
| 0.833M | 0.625M | 5.84 | more than 2000 bp |

Accordingly, according to above the result of experiments, when the concentration of chaotropic salt is fixed (concentration is 0.833M), by using monovalent salt (LiCl) with low concentration, the bigger range (include of large-size and small-szie) of nucleic acid fragments can be isolated. When the concentration of monovalent salt increasingly high, just the large-size nucleic acid fragements can be isolated. Thus, the user can select the concentration of the monovalent salt based on the desired fragments size of nucleic acid to be isolated. Further, the pH value of mixing solution (chaotropic salt and monovalent salt) is not affected by the concentration level of the monovalent salt. That is, the pH value of the mixing solution is not a mainly factor to isolate the nucleic acid fragement of specific size in the method according to the present invention.
2. Four different monovalent cation ions are measured with acetate as anion.

The DNA standard solutions for tests are prepared according to the method of the first experiment. After completed mixing of 40µL DNA standard solution and respective 200µL binding buffer solutions in different conditions, the filtration of the nucleus acids is processed by the first experiment. The compositions of the final mixing solution are shown in Table 2-3. The final concentration of GuSCN is fixed to be 0.8333M and respectively added with acetate (OAc⁻) as anion and salts of Li⁺, Na⁺, K⁺, and NH₄⁺ as cations, respectively. The final concentrations are 0.104M, 0.208M, 0.3125M, 0.416M, 0.52M, and 0.625M.

**Table 2-3: the final compositions of the final mixing solutions used in this experiment**

| Condition | Conc. Of GuSCN | Conc. Of XOAc |
|---|---|---|
| 1 | 0.833M | 0.104M |
| 2 | 0.833M | 0.208M |
| 3 | 0.833M | 0.3125M |
| 4 | 0.833M | 0.416M |
| 5 | 0.833M | 0.52M |
| 6 | 0.833M | 0.625M |

| | | |
|---|---|---|
| Note: X is Li, Na, K or NH₄ | | |

The products from nucleic acid filtration under the combined buffer solutions added with the compositions from aforementioned condition 1 to condition 6 are treated by gel electrophoresis to have results shown in Fig. 2B-Fig. 2E, and from table 2-4 to table 2-7.

Fig. 2B shows the isolation degrees of nucleic acid fragments under 0.833M GuSCN and LiOAc in different concentrations as the binding buffer solutions. The pH values of the final mixing solution under various conditions are measured to be from 7.77 to 7.90. Depicted in Fig. 2B, the size of nucleic acid fragment larger than 500 bp may be filtrated out in 0.104M LiOAc. That is, the size of nucleic acid fragments smaller than 500 bp can be eliminated because they do not bind the solid carrier. Next, the size of nucleic acid fragment larger than 5000 bp may be filtrated out in 0.208M LiOAc. That is, the size of nucleic acid fragments smaller than 5000 bp can be eliminated because they do not bind the solid carrier. Next, there is no any nucleic acid fragment left when the concentrations of LiOAc are respectively 0.3125M, 0.416M, 0.52M, and 0.625M. That is, there is no any nucleic acid fragment to be left by the solid carrier.The associations between the concentrations of LiOAc and the filtration of the nucleic acid fragment are shown in Table 2-4.

**Table 2-4: the associations between the concentrations of LiOAc and the sizes of the filtrated nucleic acid fragments**

| Conc. Of GuSCN | Conc. Of LiOAc | pH value of final mixing solution | Size of preserved nucleic acid fragment |
|---|---|---|---|
| 0.833M | 0.104M | 7.77 | more than 500 bp |
| 0.833M | 0.208M | 7.82 | more than 5000 bp |
| 0.833M | 0.3125M | 7.88 | -- |
| 0.833M | 0.416M | 7.83 | -- |
| 0.833M | 0.52M | 7.90 | -- |
| 0.833M | 0.625M | 7.84 | -- |

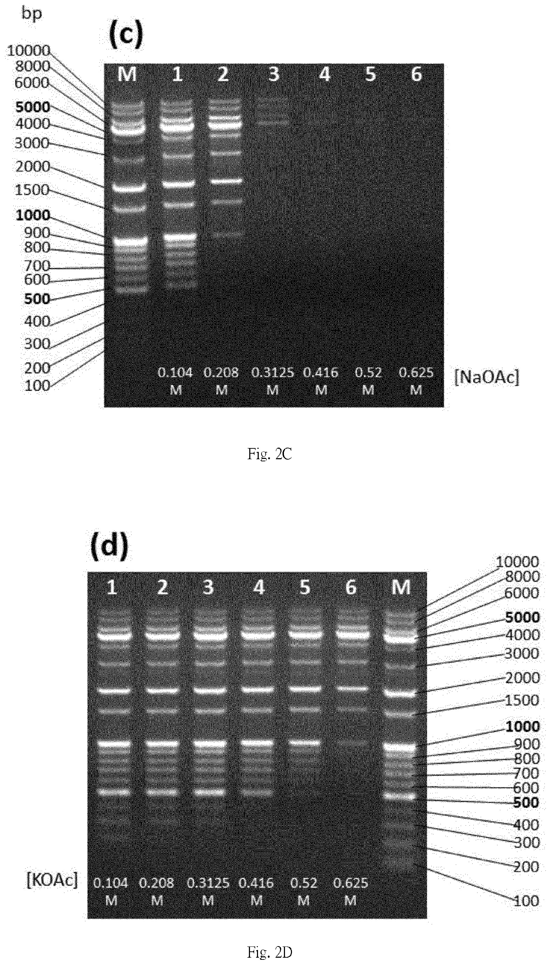

Fig. 2C shows the isolation degrees of nucleic acid fragments under 0.833M GuSCN and sodium acetate (NaOAc) in different concentrations as the binding buffer solutions. The pH values of the final mixing solution under various conditions are measured to be from 7.76 to 7.90. Depicted in Fig. 2C, the nucleic acid fragment larger than 500 bp may be filtrated out in 0.104M NaOAc. That is, the size of nucleic acid fragments smaller than 500 bp can be eliminated because they do not bind the solid carrier. Next, the size of nucleic acid fragment larger than 1000 bp may be filtrated out in 0.208M NaOAc. That is, the size of nucleic acid fragments smaller than 1000 bp can be eliminated because they do not bind the solid carrier. Next, there is no any nucleic acid fragment left when the concentrations of NaOAc are respectively 0.3125M, 0.416M, 0.52M, and 0.625M. That is, there is no any nucleic acid fragment to be left by the solid carrier. The associations between the concentrations of NaOAc and the filtration of the nucleic acid fragment are shown in Table 2-5.

**Table 2-5: the associations between the concentrations of NaOAc and the sizes of the filtrated nucleic acid fragments**

| Conc. Of GuSCN | Conc. Of NaOAc | pH value of final mixing solution | Size of preserved nucleic acid fragment |
|---|---|---|---|
| 0.833M | 0.104M | 7.76 | More than 500 bp |
| 0.833M | 0.208M | 7.80 | More than 1000 bp |
| 0.833M | 0.3125M | 7.88 | -- |
| 0.833M | 0.416M | 7.83 | -- |
| 0.833M | 0.52M | 7.90 | -- |
| 0.833M | 0.625M | 7.84 | -- |

Fig. 2D shows the isolation degrees of nucleic acid fragments under 0.833M GuSCN and KOAc in different concentrations as the binding buffer solutions. The pH values of the final mixing solutions under various conditions are measured to be from 7.88 to 8.03. Depicted in Fig. 2D, the size of nucleic acid fragment larger than 200 bp may be filtrated out in 0.104M KOAc. That is, the size of nucleic acid fragments smaller than 200 bp can be eliminated because they do not bind the solid carrier. Next, the size of nucleic acid fragment larger than 300 bp may be filtrated out in 0.208M KOAc. That is, the size of nucleic acid fragments smaller than 300 bp can be eliminated because they do not bind the solid carrier. Next, the size of nucleic acid fragment larger than 400 bp may be filtrated out in 0.3125M KOAc. That is, the size of nucleic acid fragment smaller than 400 bp can be eliminated because they do not bind the solid carrier. Next, the size of nucleic acid fragment larger than 500 bp may be filtrated out in 0.416M and 0.52M KOAc. That is, the size of nucleic acid fragments smaller than 500 bp can be eliminated because they do not bind the solid carrier. Next, the size of nucleic acid fragment larger than 1000 bp may be filtrated out in 0.625M KOAc. That is, the size of nucleic acid fragments smaller than 1000 bp can be eliminated because they do not bind the solid carrier. The associations between the concentrations of KOAc and the filtration of the nucleic acid fragment are shown in Table 2-6.

**Table 2-6: the associations between the concentrations of KOAc and the sizes of the filtrated nucleic acid fragments**

| Conc. Of GuSCN | Conc. Of KOAc | pH value of final mixing solution | Size of preserved nucleic acid fragment |
|---|---|---|---|
| 0.833M | 0.104 M | 8.01 | more than 200 bp |
| 0.833M | 0.208 M | 7.95 | more than 300 bp |
| 0.833M | 0.3125 M | 7.88 | more than 400 bp |
| 0.833M | 0.416 M | 8.03 | more than 500 bp |
| 0.833M | 0.52 M | 7.92 | more than 500 bp |
| 0.833M | 0.625 M | 7.90 | more than 1000 bp |

Fig. 2E shows the isolation degrees of nucleic acid fragments under 0.833M GuSCN and ammonium acetate (NH₄OAc) in different concentrations as the final mixing solutions. The pH values of the final mixing solutions under various conditions are measured to be from 6.58 to 6.68. Depicted in Fig. 2E, the size of nucleic acid fragment larger than 200 bp may be filtrated out in 0.104M NH₄OAc. That is, the size of nucleic acid fragments smaller than 200 bp can be eliminated because they do not bind the solid carrier. Next, the size of nucleic acid fragment larger than 300 bp may be filtrated out in 0.208M NH₄OAc. That is, the size of nucleic acid fragments smaller than 300 bp can be eliminated because they do not bind the solid carrier. Next, the size of nucleic acid fragment larger than 400 bp may be filtrated out in 0.3125M NH₄OAc. That is, the size of nucleic acid fragments smaller than 400 bp can be eliminated because they do not bind the solid carrier. Next, the size of nucleic acid fragment larger than 500 bp may be filtrated out in 0.416M NH₄OAc. That is, the size of nucleic acid fragments smaller than 500 bp can be eliminated because they do not bind the solid carrier. Next, the size of nucleic acid fragment bigger than 1000 bp may be filtrated out in 0.52M NH₄OAc. That is, the size of nucleic acid fragments smaller than 1000 bp can be eliminated because they do not bind the solid carrier. Next, the size of nucleic acid fragment larger than 2000 bp may be filtrated out in 0.625M NH₄OAc. That is, the size of nucleic acid fragments smaller than 2000 bp can be eliminated because they do not bind the solid carrier. The associations between the concentrations of NH₄Oac and the filtration of the nucleic acid fragment are shown in Table 2-7.

**Table 2-7: the associations between the concentrations of NH₄OAc and the sizes of the filtrated nucleic acid fragments**

| Conc. Of GuSCN | Conc. Of NH₄OAc | pH value of final mixing solution | Size of preserved nucleic acid fragment |
|---|---|---|---|
| 0.833M | 0.104M | 6.58 | more than 200 bp |
| 0.833M | 0.208M | 6.66 | more than 300 bp |
| 0.833M | 0.3125 M | 6.60 | more than 400 bp |
| 0.833M | 0.416 M | 6.68 | more than 500 bp |
| 0.833M | 0.52 M | 6.64 | more than 1000 bp |
| 0.833M | 0.625 M | 6.59 | more than 2000 bp |

From experiment results shown in Tables 2-4 to Table 2-7 and Fig. 2B to Fig. 2E, there are different isolation degrees on the nucleic acid fragments influenced by four salts including LiOAc, NaOAc, KOAc, and NH₄OAc in different concentrations associated with acetate as anion. It is noted that there are obviously different effects of filtration on the nucleic acid fragmentsin different sizes for four different monovalent cations.
3. The influence of NH₄OAc in different concentrations on the selection of the nucleic acid fragment is measured with NaI as monovalent salt.

The DNA standard solutions for tests are prepared according to the method of the first experiment. After completed mixing of 40µL DNA standard solution and respective 200µL binding buffer solutions in different conditions, the filtration of the nucleus acids is processed by the first experiment. The compositions of the final mixing solution are shown in Table 2-8. The final concentration of NaI is fixed to be 3M and respectively added with 0.104M, 0.208M, 0.3125M, 0.416M, 0.52M, and 0.625M NH₄OAc.

Fig. 2F shows the isolation degrees of nucleic acid fragments under the final mixing solution including 3M NaI and ammonium acetate (NH₄OAc) in different concentrations as the binding buffer solutions. The pH values of the final mixing solutions under various conditions are measured to be from 6.70 to 6.88. Depicted in Fig. 2F, the size of nucleic acid fragment larger than 500 bp may be filtrated out in 0.104M and 0.208M NaOAc. That is, the size of nucleic acid fragments smaller than 500 bp can be eliminated because they do not bind the solid carrier. Next, the size of nucleic acid fragment larger than 1000 bp may be filtrated out in 0.3125M, 0.416M, 0.52M, and 0.625M NaOAc, respectively. That is, the size of nucleic acid fragments smaller than 1000 bp can be eliminated because they do not bind the solid carrier. The associations between the concentrations of NaOAc and the filtration of the nucleic acid fragment in the final mixing solution which NaI is added into to be as chaotropic salt are shown in Table 2-8.

**Table 2-8: the associations between the concentrations of NH₄OAc and the sizes of the filtrated nucleic acid fragment in the final mixing solution which NaI is added into to be as chaotropic salt**

| condition | Conc. Of NaI | Conc. Of NH₄OAc | pH values of final mixing solution | Sizes of preserved nucleic acid |
|---|---|---|---|---|
| 1 | 3M | 0.104M | 6.83 | more than 500 bp |
| 2 | 3M | 0.208M | 6.76 | more than 500 bp |
| 3 | 3M | 0.3125M | 6.88 | more than 1000 bp |
| 4 | 3M | 0.416M | 6.70 | more than 1000 bp |
| 5 | 3M | 0.52M | 6.75 | more than 1000 bp |
| 6 | 3M | 0.625M | 6.81 | more than 1000 bp |

From experiment results shown in Table 2-8 and Fig. 2F, there are different isolation degrees on the nucleic acid fragments influenced by the final mixing solution including NaI as the chaotropic salt associated with NH₄OAc in different concentrations. It is noted that there are obviously different effects of filtration on the nucleic acid fragments in different sizes for NH₄OAc in different concentrations.

From the results of the second experiment, small-size nucleic acid fragments to be more and more difficultly caught increase with the concentrations of specific monovalent salt associated with same concentration of chaotropic salt, and vice versa. When the concentration of the specific monovalent salt is low, the bigger range (includes large-size and small-size) of nucleic acid fragments is bound on the solid carrier. With the increase of the concentration of specific monovalent salt, the specific monovalent salt merely bound the large-size nucleic acid fragments on the solid carrier but the small-size nucleic acid fragment is isolated from the solid carrier and filtered into the flow-through. Similar results happen under four monovalent cations (Li⁺, Na⁺, K⁺, NH⁴⁺) associated with fixed anion such as LiCl or acetate. It is especially obvious that acetate is as anion salt to have obvious differences in the filtration of the nucleic acid fragments in different sizes. The selection of the various monovalent cation ions is different for the nucleic acid fragment size. Furthermore, by actually measuring the pH values of the mixing solutions, the pH values of the mixing solutions are found to be varied within a narrow range. In this regard, the characteristic that the various nucleic acid fragments sizes can be selectively filtrated by the chaotropic salt and monovalent salts does not correlate with the pH values of the mixing solutions.

The third experiment discusses the influences of same chaotropic salts and crowding agents in various types and concentrations over the sizes of selected nucleic acid fragment.
1. The filtration on the sizes of the nucleic acid fragments is influenced by the crowding agent in different types and concentrations.

The DNA standard solutions for tests are prepared according to the method of the first experiment. After completed mixing of 40µL DNA standard solution and respective 200µL binding buffer solutions in different conditions, the filtration of the nucleus acids is processed by the first experiment. The compositions of the final mixing solution are shown in Table 3-2 and Table 3-3. The final concentration of GuSCN is fixed to be 0.52M and respectively added with ethanolin the final concentrations of 2.5%, 5%, 10%, 15% or 20%, or isopropanol in the final concentrations of 2.5%, 5%, 10%, 15% or 20%, respectively. The products from nucleic acid filtration under the final mixing solutions from condition 1 to condition 11 are treated by gel electrophoresis to have results shown in Fig. 3A, Fig. 3B, table 3-1, and table 3-2.

Fig. 3A shows the isolation degrees of nucleic acid fragments under 0.52M GuSCN and ethanol in different concentrations as the final mixing solutions. The pH values of the final mixing solution under various conditions are measured to be from 6.04 to 6.22. Depicted in Fig. 3A, the size of nucleic acid fragment larger than 3000 bp may be filtrated out in 2.5% ethanol. That is, the size of nucleic acid fragments smaller than 3000 bp can be eliminated because they do not bind the solid carrier. Next, the size of nucleic acid fragment larger than 1000 bp may be filtrated out in 5% ethanol. That is, the size of nucleic acid fragments smaller than 1000 bp can be eliminated because they do not bind the solid carrier. Next, the size of nucleic acid fragment larger than 500 bp may be filtrated out in 10% ethanol. That is, the size of nucleic acid fragments smaller than 500 bp can be eliminated because they do not combine with the solid carrier. Next, the size of nucleic acid fragment larger than 300 bp may be filtrated out in 15% ethanol. That is, the size of nucleic acid fragments smaller than 300 bp can be eliminated because they do not bind the solid carrier. Next, the size of nucleic acid fragments larger than 200 bp may be filtrated out in 20% ethanol. That is, the size of nucleic acid fragments smaller than 200 bp can be eliminated because they do not bind the solid carrier. The associations between the concentrations of ethanol and the filtration of the nucleic acid fragment are shown in Table 3-1.

**Table 3-1: the associations between the concentrations of ethanol and the sizes of the nucleic acid fragment**

| condition | Conc. of GuSCN | Conc. Of ethanol | pH value of final mixing solution | Sizes of preserved nucleic acid fragment |
|---|---|---|---|---|
| 1 | 0.52M | 2.5% | 6.07 | more than 3000 bp |
| 2 | 0.52M | 5% | 6.10 | more than 1000 bp |
| 3 | 0.52M | 10% | 6.13 | more than 500 bp |
| 4 | 0.52M | 15% | 6.04 | more than 300 bp |
| 5 | 0.52M | 20% | 6.22 | more than 200 bp |

Fig. 3B shows the isolation degrees of nucleic acid fragments under 0.52M GuSCN and isopropanol in different concentrations as the final mixing solution. The pH values of the final mixing solution under various conditions are measured to be from 5.79 to 6.01. Depicted in Fig. 3B, the size of nucleic acid fragment larger than 2000 bp may be filtrated out in 2.5% isopropanol. That is, the size of nucleic acid fragments smaller than 2000 bp can be eliminated because they do not bind the solid carrier. Next, the size of nucleic acid fragment larger than 1000 bp may be filtrated out in 5% isopropanol. That is, the size of nucleic acid fragments smaller than 1000 bp can be eliminated because they do not bind the solid carrier. Next, the size of nucleic acid fragment larger than 500 bp may be filtrated out in 10% isopropanol. That is, the size of nucleic acid fragments smaller than 500 bp can be eliminated because they do not bind the solid carrier. Next, the size of nucleic acid fragment larger than 300 bp may be filtrated out in 15% isopropanol. That is, the size of nucleic acid fragments smaller than 300 bp can be eliminated because they do not bind the solid carrier. Next, the size of nucleic acid fragment larger than 200 bp may be filtrated out in 20% isopropanol. That is, the size of nucleic acid fragments smaller than 200 bp can be eliminated because they do not bind the solid carrier. The associations between the concentrations of isopropanol and the filtration of the nucleic acid fragment are shown in Table 3-2.

**Table 3-2: the associations between the concentrations of isopropanol and the sizes of the nucleic acid fragment**

| condition | Conc. of GuSCN | Conc. Of isopropanol | pH value of final mixing solution | Sizes of preserved nucleic acid fragment |
|---|---|---|---|---|
| 6 | 0.52M | 2.5% | 5.85 | More than 2000 bp |
| 7 | 0.52M | 5% | 5.88 | More than 1000 bp |
| 8 | 0.52M | 10% | 5.79 | More than 500 bp |
| 9 | 0.52M | 15% | 6.01 | More than 300 bp |
| 10 | 0.52M | 20% | 5.98 | More than 200 bp |

From the experiment results shown in Table 3-1, Table 3-2, Fig. 3A and Fig. 3B, there are different filtrations on the sizes of the nucleic acid fragments made by ethanol and isopropanol of different concentrations. The smaller nucleic acid fragments to be more and more easily caught increase with the concentrations of alcohols, and vice versa.
2. Polyethylene glycol in different concentrations and molecular weights causes influence on the filtration of the nucleic acid fragments.

The DNA standard solutions for tests are prepared according to the method of the first experiment. After completed mixing of 40µL DNA standard solution and respective 200µL binding buffer solution in different conditions, the filtration of the nucleus acids is processed by the first experiment. The compositions of the final mixing solution are shown in Table 3-3 and Table 3-4. The final concentration of GuSCN is fixed to be 0.6M and respectively added with 10%, 15% and 20% PEG4000 or 10%, 15% and 20% PEG8000. The products from nucleic acid filtration under the final mixing solution from condition 12 to condition 17 are treated by gel electrophoresis to have results shown in Fig. 3C, table 3-3 and table 3-4.

Fig. 3C shows the isolation degrees of nucleic acid fragments under 0.6M GuSCN and PEG4000 in different concentrations as the binding buffer solutions. The pH values of the binding buffer solutions under various conditions are measured to be from 5.83 to 5.92. Depicted in Fig. 3C, the size of nucleic acid fragment larger than 1000 bp may be filtrated out in 10% PEG4000. That is, the size of nucleic acid fragments smaller than 1000 bp can be eliminated because they do not bind the solid carrier. Next, the size of nucleic acid fragment larger than 100 bp may be filtrated out in 15% and 20% PEG4000. That is, the size of nucleic acid fragments smaller than 100 bp can be eliminated because they do not bind the solid carrier. The associations between the concentrations of PEG4000 in the final mixing solutions and the filtration of the nucleic acid fragment are shown in Table 3-3.

**Table 3-3: the associations between the concentrations of PEG4000 and the sizes of the filtrated nucleic acid fragment**

| condition | Conc. of GuSCN | Conc. Of PEG4000 | pH value of final mixing solution | Sizes of preserved nucleic acid fragment |
|---|---|---|---|---|
| 12 | 0.6M | 10% | 5.86 | more than 1000 bp |
| 13 | 0.6M | 15% | 5.92 | More than 100 bp |
| 14 | 0.6M | 20% | 5.83 | More than 100 bp |

Fig. 3C also shows the isolation degrees of nucleic acid fragments under 0.6M GuSCN and PEG8000 in different concentrations as the binding buffer solutions. The pH values of the binding buffer solutions under various conditions are measured to be from 5.83 to 5.98. Depicted in Fig. 3C, the size of nucleic acid fragment larger than 500 bp may be filtrated out in 10% PEG8000. That is, the size of nucleic acid fragments smaller than 500 bp can be eliminated because they do not bind the solid carrier. Next, the size of nucleic acid fragmentlarger than 100 bp may be filtrated out in 15% and 20% PEG8000. That is, the size of nucleic acid fragments smaller than 100 bp can be eliminated because they do not bind the solid carrier. The associations between the concentrations of PEG8000 in the final mixing solutions and the filtration of the nucleic acid fragment are shown in Table 3-4.

**Table 3-4: the associations between the concentrations of PEG8000 and the sizes of the filtrated nucleic acid fragment**

| condition | Conc. of GuSCN | Conc. Of PEG8000 | pH value of final mixing solution | Sizes of preserved nucleic acid fragment |
|---|---|---|---|---|
| 15 | 0.6M | 10% | 5.98 | More than 500 bp |
| 16 | 0.6M | 15% | 5.83 | More than 100 bp |
| 17 | 0.6M | 20% | 5.90 | More than 100 bp |

From the experiment results shown in Table 3-3, Table 3-4, Fig. 3C, there are different filtrations on the sizes of the nucleic acid fragments made by polyethylene glycol of different concentrations and molecular weights. Under the same chaotropic salt concention (GuSCN), the smaller nucleic acid fragments to be more and more easily caught increase with the concentrations of alcohols, and vice versa. The larger molecular weight of the polyethylene glycolis easilier to catch the smaller nucleic acid fragments.

From the results of the third experiment, there are different filtration results on the sizes of the nucleic acid fragments by ethanol and isopropanol of different concentrations or polyethylene glycol of different concentrations and molecular weights. The more the concentrations or the molecular weights of the crowding agent increases, the easilier the small-size nucleic acid fragments can be caught, and vice versa. Furthermore, by actually measuring the pH values of the mixing solutions, the pH values of the mixing solutions are found to be varied within a narrow range. In this regard, the characteristic that the nucleus acid fragments in different sizes can be selectively filtrated by the chaotropic salt of different concentrations associated with the crowding agent does not correlate with the pH values of the mixing solutions.

The fourth experiment discusses the influences of solutions including the chaotropic salts, monovalent salts and the crowding agents over the specific nucleic acid fragment.

### 1. Spin column is used as solid carrier

The DNA standard solutions for tests are prepared according to the method of the first experiment. After completed mixing of 40µL DNA standard solution and respective 60µL binding buffer solutions of A1, B1 and C1 to form the mixing solutions AM1, BM1 and CM1. The pH values of the mixing solutions AM1, BM1 and CM1 are respectively 6.66, 6.63 and 6.62. The mixing solutions AM1, BM1 and CM1 are respectively added into the first spin column and centrifuged at 13000 rpm for one minute to acquire filtrates AF1, BF1 and CF1 after the first filtration. The filtrates AF1, BF1 and CF1 are respectively added with 100µL, 0.9M GuSCN to form mixing solutions AM2, BM2 and CM2. The pH values of the mixing solutions AM2, BM2 and CM2 after completed mixing are respectively 6.54, 6.61 and 6.53. The mixing solutions AM2, BM2 and CM2 are respectively added into the second spin column and centrifuged at 13000rpm for one minute to acquire filtrates AF2, BF2 and CF2 after the second filtration. The filtrates AF2, BF2 and CF2 are respectively added with 40µL, 20µL and 10µL 100% isopropanol to form to form mixing solutions AM3, BM3 and CM3. The pH values of the mixing solutions AM3, BM3 and CM3 after completed mixing are respectively 6.60, 6.56 and 6.55. The mixing solutions AM3, BM3 and CM3 are respectively added into the third spin colunm and centrifuged at 13000 rpm for one minute to remove filtrates after the third filtration. The final concentrations and the pH values of compositions in various mixing solutions are listed in Table 4-1.

**Table 4-1: the final concentrations and the pH values of compositions in various mixing solutions are listed in Table 4-1**

| solution No. | the final concentrations of compositions in various mixing solutions | pH value of compositions in mixing solution |
|---|---|---|
| AM1 | 0.9M GuSCN and 0.7M NH₄OAc | 6.66 |
| AM2 | 0.9M GuSCN and 0.35M NH₄OAc | 6.54 |
| AM3 | 0.75M GuSCN, 0.29M NH₄OAc and 16.6% isopropanol | 6.60 |
| BM1 | 0.9M GuSCN and 0.5M NH₄OAc | 6.63 |
| BM2 | 0.9M GuSCN and 0.25M NH₄OAc | 6.61 |
| BM3 | 0.82M GuSCN, 0.227M NH₄OAc and 9.1% isopropanol | 6.56 |
| CM1 | 0.9M GuSCN and 0.3M NH₄OAc | 6.62 |
| CM2 | 0.9M GuSCN and 0.15M NH₄OAc | 6.53 |
| CM3 | 0.875M GuSCN, 0.143M NH₄OAc and 4.76% isopropanol | 6.55 |

The first, second and third spin column are washed by adding 60% ethanol and centrifuged in 13000 rpm for one minute, and then added with 40µL pure water to be centrifuged in 13000 rpm for one minute to respectively acquire the first filtrated products AE1, BE1, CE1, the second filtrated products AE2, BE2 and CE2, and the third filtrated products AE3, BE3 and CE3. These products from are treated by gel electrophoresis to have results shown in Fig.4 and Table 4-2.

**Table 4-2: the sizes of the filtrated nucleic acid fragment from the various mixing solutions**

| Product No. | sizes of preserved nucleic acid fragment |
|---|---|
| AE1 | More than 3000 bp |
| AE2 | Between 2000 and 600 bp |
| AE3 | Between 500 and 100 bp |
| BE1 | More than 1000 bp |
| BE2 | Between 1000 and 500 bp |
| BE3 | Between 500 and 200 bp |
| CE1 | More than 500 bp |
| CE2 | 500 bp |
| CE3 | Between 300 and 200 bp |

According to Table 4-1, Table 4-2 and the result of above experiments, AM1, AM2, BM1, BM2, CM1, and CM 2 under the same concentration of chaotropic salt, the different concentration of monovalent salt can preserve the different size nucleic acid fragment. As aforementioned, the high concentraiton of monovalent salt can preserve the small-size nucleic acid fragment; on the contrary, the lower concentration of monovalent salt can preserve the large nucleic acid fragment range, in which large nucleic acid fragment range means the nucleic acid may include large-size, middle size and/or small size fragment.

### 2. ceramic magnetic beads as the solid carrier

A DNA standard solution for tests is prepared according to the first experiment. A 40µL DNA standard solution is mixed respectively with 60µL binding buffer solution D1 (1.5M GuSCN), 0.8M NH₄OAc and solution E1 (2.5M GuSCN and 2.5M NH₄OAc) completed to respectively form mixing solutions, DM1 and EM1. The pH values of the mixing solutions DM1 and EM1 are respectively 6.53 and 6.60. The mixing solutions, DM1 and EM1 are respectively mixed with a first magnetic beads (Magnetic beads MF-SIL-5024, MagQu, Xinbei City, Taiwan) by oscillating for five minutes and then positioned on a magnetic base for adsorbing magnetic beads for one minute to acquire upper clean liquid DS1 and ES1 after the first filtration. Next, the mixing solutions DM2 and EM2 by respectively mixing the upper clean liquid DS1 with 20µL, 100% isopropanol and the upper clean liquid ES1 with 100µL, 2.5M GuSCN. The pH values of the mixing solutions DM2 and EM2 are respectively 6.60 and 6.54. The mixing solutions DM2 and EM2 are respectively mixed with second magnetic beads by oscillating for five minutes and then positioned on a magnetic base for adsorbing magnetic beads for one minute to acquire upper clean liquid DS2 and ES2 after the second filtration. Next, the mixing solutions DM3 and EM3 by respectively mixing the upper clean liquid DS2 with 120µL, 2.5M GuSCN and the upper clean liquid ES2 with 200µL, 100% isopropanol. The pH values of the mixing solutions DM3 and EM3 are respectively 6.51 and 6.61. The final concentrations and pH values of compositions in the various mixing solutions are listed in Table 4-3.

**Table 4-3: final concentrations and pH values of compositions in the various mixing solutions**

| Solution No. | final concentrations of compositions in the various mixing solutions | pH values of compositions in the various mixing solutions |
|---|---|---|
| DM1 | 0.9M GuSCN and 0.48M NH₄OAc | 6.53 |
| DM2 | 0.75M GuSCN, 0.4M NH4OAc and 16.67% isopropanol | 6.60 |
| DM3 | 1.625M GuSCN, 0.2M NH₄OAc and 10% isopropanol | 6.51 |
| EM1 | 1.5M GuSCN and 1.5M NH₄OAc | 6.60 |
| EM2 | 2M GuSCN and 0.75M NH₄OAc | 6.54 |
| EM3 | 1M GuSCN, 0.325M NH₄OAc and 50% isopropanol | 6.61 |

The first, second and third magnetic beads are respectively rinsed by adding 60% ethanol and oscillating 30 seconds, and then positioned onto the magnetic base for one minute for the removal of upper clean liquids. The first residue is dried at room temperature for five minutes, eluted by adding 40µL pure aqureous solution and then again oscillating for one minute, positioned on the magnetic base for one minute, and filtrated out upper clean liquids to acquire the first filtrated products DE1 and EE1, the second filtrated products DE2 and EE2, and the third filtrated products DE3 and EE3. These filtrated products are treated by gel electrophoresis to be measured listed in Fig. 5 and Table 4-4.

**Table 4-4: the sizes of filtrated nucleic acid fragments in various mixing solutions**

| product No. | Sizes of filtrated nucleic acid fragment |
|---|---|
| DE1 | more than 1000 bp |
| DE2 | Between 1000 and 200 bp |
| DE3 | 100 bp |
| EE1 | more than 1000 bp |
| EE2 | Between 1000 and 200 bp |
| EE3 | 100 bp |

According to Table 4-3, Table 4-4 and the results of above experiments, DM2 and DM3 shows that when the concentration of chaotropic salt is increased, the concnetration of monovalent salt is decreased and the concentration of crowding agent is drcreased, the filtered nucleic acid fragement is getting smaller.

From the results of Fig. 4 and Fig. 5, the specific sizes of nucleic acid fragment can be acquired by appling a most suitable solution condition, the concentration of chaotropic salt, the concention of monovalent salt and/or the concentration of crowding agent can isolate the nucleic acid fragment of a specific size, no matter spin column or magnetic beads is associated with. The method according to the present invention also works for the isolation of small-size nucleic acid fragments. Even for the nucleic acid fragments fall within a same period, the filtration or isolation can be achieved by selecting chaotropic salts in different concentrations, monovalent salts and crowding agents. Thus, various combinations can be adjusted according to the requirements of environment in operation. According to the brightness comparison of the filtrated nucleic acid fragments in electropherograms with the ones in the standard solutions, the recovery rate provided by the method according to the present invention is high, and the method of the present invention is suitable for the isolation, purification and recovery of different nucleic acid fragments. Furthermore, according to the pH measurement of the mixing solutions, the variation of pH values is found to be very narrow. The characteristic that the nucleus acid fragments in different sizes can be selectively filtrated by the chaotropic salts, the monovalent salts and the crowding agents in different concentrations does not correlate with the pH values of the mixing solutions.

Accordingly, by adjusting three different factors aforementioned and going through once, twice or three times filtrations, any required smaller or specific nucleic acid fragments can be isolated from a sample, which is fast and efficient purification and filtration for nucleic acid fragments. On the other hand, the filtration or isolation according to the present disclosure does not need any modified solid carrier and is suitable for base pairs in the fragment sizes of 100 to 5000 bp. Accordingly the selection and usefulness for the filtration of nucleic acid are improved.

## Claims

1. A method for selective isolation of nucleic acid, comprising:
(a) mixing a nucleic acid sample and a binding buffer solution to form a mixing solution, wherein the nucleic acid sample has a nucleic acid fragment of a specific size, and the mixing solution has a chaotropic salt and one or two of a monovalent salt and a crowding agent, wherein the chaotropic salt is guanidine hydrochloride (GuHCl), guanidine thiocyanate (GuSCN), sodium iodide (NaI) or sodium perchlorate (NaClO₄), wherein the monovalent salt is lithium chloride (LiCl), lithium acetate (LiOAc), sodium acetate (NaOAc), potassium acetate (KOAc) or ammonium acetate (NH₄OAc), wherein the crowding agent is ethanol, isopropanol or polyethylene glycol, wherein when the concentration of monovalent salt is high, the large-size nucleic acid fragment is bound to the solid carrier and when the concentration of monovalent salt is low, the bigger range which includes large-size and small-size of nucleic acid fragments are bound to the solid carrier;
(b) passing the mixing solution through a solid carrier to make the nucleic acid fragment of the specific size isolately bind to the solid carrier;
(c) passing a washing buffer liquid through the solid carrier to remove subject that does not bind the solid carrier; and
(d) passing an aqueous solution through the solid substrate to recovery the nucleic acid fragment of the specific size,
whereby changes of pH value of the mixing solution are less than or equal to 0.4, if the concentration of guanidine hydrochloride (GuHCl) is changed from 0.6M to 4M or the concentration of guanidine thiocyanate (GuSCN) is changed from 0.6M to 4M or the concentration of sodium iodide (NaI) is changed from 2.5M to 3.5M or the concentration of sodium perchlorate (NaClO₄) is changed from 2.5M to 3.5M.

2. The method for selective isolation of nucleic acid according to claim 1, wherein the solid carrier is ceramic filter rods or ceramic magnetic beads.

3. A method for selective isolation of nucleic acid, comprising:
(a) mixing a nucleic acid sample and a first binding buffer solution to form a first mixing solution, wherein the nucleic acid sample has a nucleic acid fragment of a specific size, and the first mixing solution has a chaotropic salt, wherein the chaotropic salt is guanidine hydrochloride (GuHCl), guanidine thiocyanate (GuSCN), sodium iodide (NaI) or sodium perchlorate (NaClO₄);
(b) passing the first mixing solution through a first solid carrier to acquire a once filtrated liquid, wherein the once filtrated liquid comprises the nucleic acid fragment of the specific size;
(c) mixing the once filtrated liquid and a second binding buffer solution to form a second mixing solution, wherein the second mixing solution has the chaotropic salt, and one or two of a monovalent salt and a crowding agent, wherein the monovalent salt is lithium chloride (LiCl), lithium acetate (LiOAc), sodium acetate (NaOAc), potassium acetate (KOAc) or ammonium acetate (NH₄OAc), wherein the monovalent salt is lithium chloride (LiCl), lithium acetate (LiOAc), sodium acetate (NaOAc), potassium acetate (KOAc) or ammonium acetate (NH₄OAc), wherein the crowding agent is ethanol, isopropanol or polyethylene glycol;
(d) passing second mixing solution through a second solid carrier to make the nucleic acid fragment of the specific size isolately bind to the second solid carrier, wherein when the concentration of monovalent salt is high, the large-size nucleic acid fragment is bound to the solid carrier and when the concentration of monovalent salt is low, the bigger range which includes large-size and small-size of nucleic acid fragments are bound to the solid carrier;
(e) passing a washing buffer liquid through the second solid carrier to remove subject that does not bind to the second solid carrier; and
(f) passing an aqueous solution through the second solid carrier to recovery the nucleic acid fragment of the specific size,
whereby changes of pH value of the mixing solution are less than or equal to 0.4, if the concentration of guanidine hydrochloride (GuHCl) is changed from 0.6M to 4M or the concentration of guanidine thiocyanate (GuSCN) is changed from 0.6M to 4M or the concentration of sodium iodide (NaI) is changed from 2.5M to 3.5M or the concentration of sodium perchlorate (NaClO₄) is changed from 2.5M to 3.5M.

4. The method for selective isolation of nucleic acid according to claim 3, wherein the solid carrier is ceramic filter rods or ceramic magnetic beads.

5. A kit for selective isolation of nucleic acid, the kit having at least a binding buffer solution including a chaotropic salt, and at least one of a monovalent salt or a crowding agent, and at least a solid carrier,
**characterised in that**
the chaotropic salt is guanidine hydrochloride (GuHCl), guanidine thiocyanate (GuSCN), sodium iodide (NaI) or sodium perchlorate (NaClO₄), wherein the monovalent salt is lithium chloride (LiCl), lithium acetate (LiOAc), sodium acetate (NaOAc), potassium acetate (KOAc) or ammonium acetate (NH₄OAc), wherein the crowding agent is ethanol, isopropanol or polyethylene glycol,
wherein, when the concentration of chaotropic salt is fixed, the concentration of crowding agent is low, or the concentration of monovalent salt is higher than 0.208 M and the large-size nucleic acid fragment that is bound to the solid carrier is bigger than 300 bp or 2000 bp, or the concentration of monovalent salt is 0.625 M and the large-size nucleic acid fragment that is bound to the solid carrier is bigger than 1000 bp or 5000 bp,
and when the concentration of monovalent salt is lower than 0.208 M, the bigger range which includes large-size and small-size of nucleic acid fragments are bound to the solid carrier,
whereby a mixing solution is formed by mixing the binding buffer solution and the nucleic acid, and changes of pH value of the mixing solution are less than or equal to 0.4, if the concentration of guanidine hydrochloride (GuHCl) is changed from 0.6M to 4M or the concentration of guanidine thiocyanate (GuSCN) is changed from 0.6M to 4M or the concentration of sodium iodide (NaI) is changed from 2.5M to 3.5M or the concentration of sodium perchlorate (NaClO₄) is changed from 2.5M to 3.5M.

## Patentansprüche

1. Verfahren zur selektiven Isolierung einer Nukleinsäure, welches umfasst:
(a) Mischen einer Nukleinsäureprobe mit einer Bindepufferlösung zur Bildung einer Mischungslösung, wobei die Nukleinsäureprobe ein Nukleinsäurefragment mit einer spezifischen Größe aufweist, und wobei die Mischungslösung ein chaotropes Salz und ein oder zwei von einem monovalenten Salz und einem Ansammlungsmittel beinhaltet, wobei das chaotrope Salz Guanidinhydrochlorid (GuHCl), Guanidinthiocyanat (GuSCN), Natriumiodid (Nal) oder Natriumperchlorat (NaClO₄) ist, wobei das monovalente Salz Lithiumchlorid (LiCl), Lithiumacetat (LiOAc), Natriumacetat (NaOAc), Kaliumacetat (KOAc) oder Ammoniumacetat (NH₄OAc) ist, wobei das Ansammlungsmittel Ethanol, Isopropanol oder Polyethylenglykol ist, wobei, wenn die Konzentration des monovalenten Salzes hoch ist, ein großdimensioniertes Nukleinsäurefragment an einen festen Träger gebunden wird, und wobei, wenn die Konzentration des monovalenten Salzes gering ist, der größere Teil, welcher großdimensionierte und kleindimensionierte Nukleinsäurefragmente beinhaltet, an den festen Träger gebunden wird,
(b) Durchleiten der Mischungslösung durch einen festen Träger, um das Nukleinsäurefragment mit der spezifischen Größe isoliert an den festen Träger binden zu lassen,
(c) Durchleiten einer Waschpufferflüssigkeit durch den festen Träger, um Materie zu entfernen, die nicht an den festen Träger gebunden ist, und
(d) Durchleiten einer wässrigen Lösung durch das feste Trägermaterial zur Rückgewinnung des Nukleinsäurefragments mit der spezifischen Größe,
wobei Änderungen des pH-Werts der Mischungslösung geringer oder gleich 0,4 sind, wenn die Konzentration von Guanidinhydrochlorid (GuHCl) von 0,6 M zu 4 M geändert wird oder die Konzentration von Guanidinthiocyanat (GuSCN) von 0,6 M zu 4 M geändert wird oder die Konzentration von Natriumiodid (NaI) von 2,5 M zu 3,5 M geändert wird oder die Konzentration von Natriumperchlorat (NaClO₄) von 2,5 M zu 3,5 M geändert wird.

2. Verfahren zur selektiven Isolierung einer Nukleinsäure nach Anspruch 1, bei welchem der feste Träger keramische Filterstäbe oder keramische Magnetperlen sind.

3. Verfahren zur selektiven Isolierung einer Nukleinsäure, welches umfasst:
(a) Mischen einer Nukleinsäureprobe mit einer ersten Bindepufferlösung zur Bildung einer ersten Mischungslösung, wobei die Nukleinsäureprobe ein Nukleinsäurefragment mit einer spezifischen Größe aufweist, und wobei die erste Mischungslösung ein chaotropes Salz beinhaltet, wobei das chaotrope Salz Guanidinhydrochlorid (GuHCl), Guanidinthiocyanat (GuSCN), Natriumiodid (NaI) oder Natriumperchlorat (NaClO₄) ist,
(b) Durchleiten der ersten Mischungslösung durch einen ersten festen Träger, um eine einmal filtrierte Flüssigkeit zu erhalten, wobei die einmal filtrierte Flüssigkeit das Nukleinsäurefragment mit der spezifischen Größe beinhaltet,
(c) Mischen der einmal filtrierten Flüssigkeit mit einer zweiten Bindepufferlösung zur Bildung einer zweiten Mischungslösung, wobei die zweite Mischungslösung das chaotrope Salz und ein oder zwei von einem monovalenten Salz und einem Ansammlungsmittel beinhaltet, wobei das monovalente Salz Lithiumchlorid (LiCl), Lithiumacetat (LiOAc), Natriumacetat (NaOAc), Kaliumacetat (KOAc) oder Ammoniumacetat (NH₄OAc) ist, wobei das monovalente Salz Lithiumchlorid (LiCl), Lithiumacetat (LiOAc), Natriumacetat (NaOAc), Kaliumacetat (KOAc) oder Ammoniumacetat (NH₄OAc) ist und wobei das Ansammlungsmittel Ethanol, Isopropanol oder Polyethylenglykol ist,
(d) Durchleiten der zweiten Mischungslösung durch einen zweiten festen Träger, um das Nukleinsäurefragment mit der spezifischen Größe isoliert an den zweiten festen Träger binden zu lassen, wobei, wenn die Konzentration des monovalenten Salzes hoch ist, ein großdimensioniertes Nukleinsäurefragment an den festen Träger gebunden wird, und wobei, wenn die Konzentration des monovalenten Salzes niedrig ist, der größere Teil, welcher großdimensionierte und kleindimensionierte Nukleinsäurefragmente beinhaltet, an den festen Träger gebunden wird,
(e) Durchleiten einer Waschpufferflüssigkeit durch den zweiten festen Träger, um Materie zu entfernen, die nicht an den zweiten festen Träger gebunden ist, und
(f) Durchleiten einer wässrigen Lösung durch den zweiten festen Träger zur Rückgewinnung des Nukleinsäurefragments mit der spezifischen Größe, wobei Änderungen des pH-Werts der Mischungslösung geringer oder gleich 0,4 sind, wenn die Konzentration von Guanidinhydrochlorid (GuHCl) von 0,6 M zu 4 M geändert wird oder die Konzentration von Guanidinthiocyanat (GuSCN) von 0,6 M zu 4 M geändert wird oder die Konzentration von Natriumiodid (NaI) von 2,5 M zu 3,5 M geändert wird oder die Konzentration von Natriumperchlorat (NaClO₄) von 2,5 M zu 3,5 M geändert wird.

4. Verfahren zur selektiven Isolierung einer Nukleinsäure nach Anspruch 3, bei welchem der feste Träger keramische Filterstäbe oder keramische Magnetperlen sind.

5. Set zur selektiven Isolierung einer Nukleinsäure, wobei das Set zumindest eine Bindepufferlösung, die ein chaotropes Salz umfasst, und zumindest ein monovalentes Salz oder ein Ansammlungsmittel, und zumindest einen festen Träger beinhaltet,
**dadurch gekennzeichnet, dass**
das chaotrope Salz Guanidinhydrochlorid (GuHCl), Guanidinthiocyanat (GuSCN), Natriumiodid (NaI) oder Natriumperchlorat (NaClO₄) ist, wobei das monovalente Salz Lithiumchlorid (LiCl), Lithiumacetat (LiOAc), Natriumacetat (NaOAc), Kaliumacetat (KOAc) oder Ammoniumacetat (NH₄OAc) ist, und wobei das Ansammlungsmittel Ethanol, Isopropanol oder Polyethylenglykol ist, wobei, wenn die Konzentration des chaotropen Salzes fest ist, die Konzentration des Ansammlungsmittels gering ist oder die Konzentration des monovalenten Salzes höher als 0,208 M ist, das großdimensionierte Nukleinsäurefragment, das an den festen Träger gebunden wird, größer ist als 300 bp oder 2000 bp, oder die Konzentration des monovalenten Salzes 0,625 M beträgt und das großdimensionierte Nukleinsäurefragment, das an den festen Träger gebunden wird, größer ist als 1000 bp oder 5000 bp,
und wobei, wenn die Konzentration des monovalenten Salzes geringer als 0,208 M ist, der größere Teil, welcher großdimensionierte und kleindimensionierte Nukleinsäurefragmente beinhaltet, an den festen Träger gebunden wird, wobei eine Mischungslösung durch Mischen der Bindepufferlösung mit der Nukleinsäure gebildet wird, und wobei Änderungen des pH-Werts der Mischungslösung geringer oder gleich 0,4 sind, wenn die Konzentration von Guanidinhydrochlorid (GuHCl) von 0,6 M zu 4 M geändert wird oder die Konzentration von Guanidinthiocyanat (GuSCN) von 0,6 M zu 4 M geändert wird oder die Konzentration von Natriumiodid (NaI) von 2,5 M zu 3,5 M geändert wird oder die Konzentration von Natriumperchlorat (NaClO₄) von 2,5 M zu 3,5 M geändert wird.

## Revendications

1. Procédé d'isolement sélectif d'un acide nucléique, comprenant:
(a) le mélange d'un échantillon d'acide nucléique et d'une solution tampon de fixation pour former une solution de mélange, dans lequel l'échantillon d'acide nucléique a un fragment d'acide nucléique d'une taille spécifique, et la solution de mélange comporte un sel chaotropique et un ou deux parmi un sel monovalent et un agent de peuplement, dans lequel le sel chaotropique est le chlorhydrate de guanidine (GuHCl), le thiocyanate de guanidine (GuSCN), l'iodure de sodium (NaI) ou le perchlorate de sodium (NaClO₄), dans lequel le sel monovalent est le chlorure de lithium (LiCl), l'acétate de lithium (LiOAc), l'acétate de sodium (NaOAc), l'acétate de potassium (KOAc) ou l'acétate d'ammonium (NH₄OAc), dans lequel l'agent de peuplement est l'éthanol, l'isopropanol ou le polyéthylène glycol, dans lequel lorsque la concentration en sel monovalent est élevée, le fragment d'acide nucléique de grande taille est fixé au support solide et lorsque la concentration du sel monovalent est basse, la plus grosse plage qui inclut la grande taille et la petite taille des fragments d'acide nucléique est fixée au support solide;
(b) le passage de la solution de mélange à travers un support solide pour amener le fragment d'acide nucléique de la taille spécifique à se fixer isolément au support solide;
(c) le passage d'un liquide tampon de lavage à travers le support solide pour éliminer un sujet qui ne s'est pas fixé au support solide; et
(d) le passage d'une solution aqueuse à travers le substrat solide pour récupérer le fragment d'acide nucléique de la taille spécifique,
moyennant quoi des changements de valeur de pH de la solution de mélange sont inférieurs ou égaux à 0,4, si la concentration en chlorhydrate de guanidine (GuHCl) est passée de 0,6 M à 4 M ou que la concentration en thiocyanate de guanidine (GuSCN) est passée de 0,6 M à 4 M ou que la concentration en iodure de sodium (Nal) est passée de 2,5 M à 3,5 M ou la concentration en perchlorate de sodium (NaClO₄) est passée de 2,5 M à 3,5 M.

2. Procédé d'isolement sélectif d'un acide nucléique selon la revendication 1, dans lequel le support solide est des tiges-filtres en céramique ou des billes magnétiques en céramique.

3. Procédé d'isolement sélectif d'un acide nucléique, comprenant:
(a) le mélange d'un échantillon d'acide nucléique et d'une première solution tampon de fixation pour former une première solution de mélange, dans lequel l'échantillon d'acide nucléique a un fragment d'acide nucléique d'une taille spécifique, et la première solution de mélange comporte un sel chaotropique, dans lequel le sel chaotropique est le chlorhydrate de guanidine (GuHCl), le thiocyanate de guanidine (GuSCN), l'iodure de sodium (NaI) ou le perchlorate de sodium (NaClO₄);
(b) le passage de la première solution de mélange à travers un premier support solide pour acquérir un liquide filtré une fois, dans lequel le liquide filtré une fois comprend le fragment d'acide nucléique de la taille spécifique;
(c) le mélange du liquide filtré une fois et d'une seconde solution tampon de fixation pour former une seconde solution de mélange, dans lequel la seconde solution de mélange comporte le sel chaotropique, et un ou deux éléments parmi un sel monovalent et un agent de peuplement, dans lequel le sel monovalent est le chlorure de lithium (LiCl), l'acétate de lithium (LiOAc), l'acétate de sodium (NaOAc), l'acétate de potassium (KOAc) ou l'acétate d'ammonium (NH₄OAc), dans lequel le sel monovalent est le chlorure de lithium (LiCl), l'acétate de lithium (LiOAc), l'acétate de sodium (NaOAc), l'acétate de potassium (KOAc) ou l'acétate d'ammonium (NH₄OAc), dans lequel l'agent de peuplement est l'éthanol, l'isopropanol ou le polyéthylène glycol;
(d) le passage de la seconde solution de mélange à travers un second support solide pour amener le fragment d'acide nucléique de la taille spécifique à se fixer isolément au second support solide, dans lequel lorsque la concentration en sel monovalent est élevée, le fragment d'acide nucléique de grande taille est fixé au support solide et lorsque la concentration en sel monovalent est faible, la plus grosse plage qui inclut la grande taille et la petite taille des fragments d'acide nucléique est fixée au support solide;
(e) le passage d'un liquide tampon de lavage à travers le second support solide pour éliminer un sujet qui ne s'est pas fixé au second support solide; et
(f) le passage d'une solution aqueuse à travers le second support solide pour récupérer le fragment d'acide nucléique de la taille spécifique,
moyennant quoi des changements de valeur de pH de la solution de mélange sont inférieurs ou égaux à 0,4, si la concentration en chlorhydrate de guanidine (GuHCl) est passée de 0,6 M à 4 M ou que la concentration en thiocyanate de guanidine (GuSCN) est passée de 0,6 M à 4 M ou que la concentration en iodure de sodium (NaI) est passée de 2,5 M à 3,5 M ou la concentration en perchlorate de sodium (NaClO₄) est passée de 2,5 M à 3,5 M.

4. Procédé d'isolement sélectif d'un acide nucléique selon la revendication 3, dans lequel le support solide est des tiges-filtres en céramique ou des billes magnétiques en céramique.

5. Nécessaire d'isolement sélectif d'acide nucléique, le nécessaire comportant au moins une solution tampon de fixation incluant un sel chaotropique, et au moins l'un d'un sel monovalent ou d'un agent de peuplement, et au moins un support solide,
**caractérisé en ce que**
le sel chaotropique est le chlorhydrate de guanidine (GuHCl), le thiocyanate de guanidine (GuSCN), l'iodure de sodium (NaI) ou le perchlorate de sodium (NaClO₄), dans lequel le sel monovalent est le chlorure de lithium (LiCl), l'acétate de lithium (LiOAc), l'acétate de sodium (NaOAc), l'acétate de potassium (KOAc) ou l'acétate d'ammonium (NH₄OAc), dans lequel l'agent de peuplement est l'éthanol, l'isopropanol ou le polyéthylène glycol,
dans lequel lorsque la concentration en sel chaotropique est fixée, la concentration en agent de peuplement est basse, ou que la concentration en sel monovalent est plus élevée que 0,208 M et que le fragment d'acide nucléique de grande taille qui est fixé au support solide est plus gros que 300 pb ou 2 000 pb, ou que la concentration en sel monovalent est de 0,625 M et que le fragment d'acide nucléique de grande taille qui est fixé au support solide est plus gros que 1 000 pb ou 5 000 pb,
et lorsque la concentration en sel monovalent est plus basse que 0,208 M, la plus grosse plage qui inclut la grande taille et la petite taille de fragments d'acide nucléique est fixée au support solide,
moyennant quoi une solution de mélange est formée en mélangeant la solution tampon de fixation et l'acide nucléique, et des changements de valeur de pH de la solution de mélange sont inférieurs ou égaux à 0,4, si la concentration en chlorhydrate de guanidine (GuHCl) est passée de 0,6 M à 4 M ou que la concentration en thiocyanate de guanidine (GuSCN) est passée de 0,6 M à 4 M ou que la concentration en iodure de sodium (Nal) est passée de 2,5 M à 3,5 M ou que la concentration en perchlorate de sodium (NaClO₄) est passée de 2,5 M à 3,5 M.
